Europäisches Patentamt

European Patent Office

Office européen des brevets

(11) Publication number: **0 166 337**
**B1**

# EUROPEAN PATENT SPECIFICATION

(45) Date of publication of patent specification: 20.09.89

(51) Int. Cl.⁴: **A 61 K 7/06**

(21) Application number: **85107430.2**

(22) Date of filing: **14.06.85**

(54) Elastase-containing hair care agent.

(30) Priority: **14.06.84 JP 120718/84**

(43) Date of publication of application:
**02.01.86 Bulletin 86/01**

(45) Publication of the grant of the patent:
**20.09.89 Bulletin 89/38**

(84) Designated Contracting States:
**AT BE CH DE FR GB IT LI NL SE**

(56) References cited:
**EP-A-0 097 810**
**FR-A-1 523 250**
**GB-A-1 448 276**

**CHEMICAL ABSTRACTS, vol. 93, 1980, page
318, no. 31635k, Columbus, Ohio, US; & SU - A
- 719 630 (ALL-UNION SCIENTIFIC-RESEARCH
BIOTECHNICAL INSTITUTE; "FLORA"
INDUSTRIAL ENTERPRISES OF CONSUMER
CHEMISTRY) 05-03-1980**

(73) Proprietor: **Eisai Co., Ltd.**
**6-10, Koishikawa 4-chome Bunkyo-ku
Tokyo 112 (JP)**

(72) Inventor: **Mato, Masao**
**162-1, Azuma-cho 2-chome
Ohmiya-shi Saitama Prefecture (JP)**
Inventor: **Takeda, Yoshinori**
**3-19-3, Miyazono
Nagareyama-shi Chiba Prefecture (JP)**
Inventor: **Osawa, Shigemitsu**
**2286-12, Suehior-cho
Honjyo-shi Saitama Prefecture (JP)**

(74) Representative: **Lehn, Werner, Dipl.-Ing. et al
Hoffmann, Eitle & Partner Patentanwälte
Arabellastrasse 4
D-8000 München 81 (DE)**

## Description

This invention relates to a hair car agent for topical application comprising elastase as an effective ingredient. Elastase itself is already known. Therefore, this invention is directed to a new use of elastase for the purpose of hair care in the fields of pharmaceuticals and cosmetics. The term "hair care" as used herein must be interpreted in broad sense, namely, it means to invigorate, restore, refresh and/or stimulate the hair. The subject matter of this invention, hair care agent, must hence be distinguished from mere hair dressings which are routinely used in grooming and styling the hair.

Elastase is an enzyme which serves to singularly degrade elastin, a water-insoluble scleroprotein. Biochemically, it has been recognized to have β-lipoproteinase activity and lipoproteinlipase activity and acts to normalize lipid dysbolism in both serum and tissues. It is thus clinically used to improve serolipid disorder which is induced along with hyperlipemia and arterioschlerosis. It also acts for arterial walls *per se* to maintain and enhance their elasticity and distensibility. In other words, it has such functions that modified elastin is taken away from arterial walls, production of fresh elastin is promoted, deposition of fat on modified elastin is suppressed, and development of atheroschlerosis is inhibited. Reference may be made to the following articles 1)—6), which are written in the Japanese language except for the article 6).

1) OGAWA, Kazuro; and WAGASATO, Yasuhiro: 'Morphological Study on the Anti-atheroschlerosis Effect of Elastase", Nichiroishi, *10*, 277—292 (1973);

2) OSAWA, Akira; "Antiarterioschlerotic Effect of Elastase (Elastolytic Enzyme)", Nichinaikaishi, *59*, 20—29 (1970);

3) NAITO, Shuko; TONO, Toshio; AWABUCHI, Tsutomu; ISHIMARU, Yoshie; UESUGI Masahide; OGASAWARA, Michio; OMORI, Akimasa; KASE, Masao; KIMURA, Hitoshi; NANASATO, Yasushi; and YOKOYAMA, Saburo: "Discussion by the Double Blind Test on the Effects of Elastase for Improving Serolipid Disorder", Igaku no Ayumi, *82*, 848—859 (1972);

4) TONO, Toshio: "Treatment of Arteriosclerotic Diseases by Elastase", Rinsho to Kenkyu, *53*, 1798 (1976);

5) HASEGAWA, Motoharu; KAWASAKI, Ken; ARAI Chikao et al: "Antiarteriosclerotic Activities of Elastase", Domyaku Koka, *18*(2), 271—286 (1980); and

6) Balo, J. and Banga, I.: "The Elastolytic Activity of Pancreatic Extracts", Biochem. J., *46*, 384 (1950).

In addition to the above-mentioned application of elastase for hyperlipemia, arteriosclerosis and hypertension, it has been found that elastase can also be used effectively for diabetes and renal diseases developed along with diabetes, resulting in the development of new utility for elastase. In this concern, reference may be made to the following articles 7)—9), the first two of which are written in the Japanese language.

7) OIKAWA, Shinichi; KAKAZAKI, Masae; and GOTO, Yoshio: "Inhibition of Tylosis of Basilemma of Rat Renal Glomeruli, Caused by Spontaneous Diabetes, by Pancreatic Elastase", Igaku no Ayumi, *111*, 583—584 (1979);

8) KASEDA, Nobuhiro: ·"Effects of Elastase on Initial Renal Lesion of KK Mice Suffering from Spontaneous Diabetes — Morphological Observation by Electron Microscope", Rinsho Seijinbyo, 7, 133—139 (1977); and

9) SHIBATA, Masao, KASAI, Atsuko; KISHI, Tsunenori; KABAYASHI, Kaizo; YADUDA, Bunji; KUNO, Jyoji; and SASAKI, Minoru: "Effects of Elastase on Glomerular Lesion of N.S.Y. Mice Suffering from Spontaneous Diabetes", Nagoya J. Med. Sci., *43*, 111 (1981).

One of the inventors conducted a variety of investigations with a view toward developing new pharmaceutical utility on elastase. As a result, it was found that elastase is effective for the removal of lipid accumulated in histiocytic cells and in addition, can significantly activate aged vascular smooth muscles. Reference may be made to the following article 10) which is written in the Japanese language.

10) MATO, Masao; OKAWARA, Shigeo; SANO, Motohiko; and KURIHASHI, Kyoichi: "Study on Cleaning Cells in Brain (Fluorescent Granular Circumferential Cells) — Especially, Incorporation of Hemolipid", No to Shnkei, *34*, No. 10, 989—997 (1982).

FR—A—1 523 250 reveals cosmetic compositions against wrinkles comprising elastane.

GB—A—1 448 296 includes a therapeutic composition for the treatment especially of conditions characterised by serum hyperlipidaemia. The above medicament comprises an esterically coated solid dosage unit including elastase and a pharmaceutical carrier therefor.

One of the inventors proceeded with a further investigation. As a result, it came to his attention that elastase shows inhibitory effects against depilation which is developed as a side effect upon administration of adriamycin, an anticarcinomelcositic agent. In an experiment on aged animals, it was also observed that an elastase-applied group generally had fine coats of hair and developed less effluvium capillorum. Such findings were unexpected findings which were not inferable or conceivable from the previous findings on elastase. Further experiments have then been carried out on the basis of such findings, leading to completion of this invention.

As apparent from the foregoing, an object of this invention is to provide a hair care agent for topical application comprising elastase as an effective ingredient.

The hair care agent of this invention is effective in promoting the growth of hair.

The above and other objects, features and advantages of this invention will become apparent from the following description and the appended claims, taken in conjunction with the accompanying drawings.

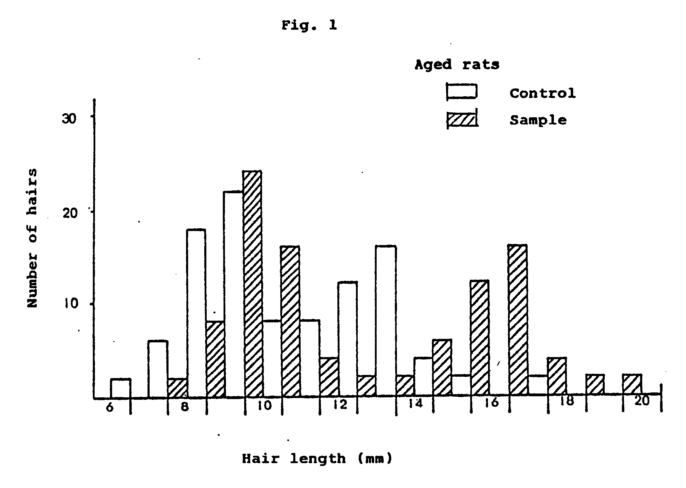

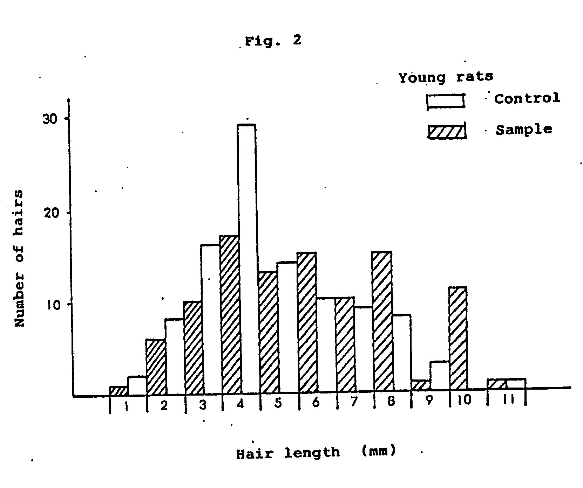

Figs. 1 and 2 are histograms each of which shows the distribution in length of hairs on the backs of rats tested in Experiment 1, which will be described herein.

Industrially, elastase is extracted and provided using swine pancreas. As elastase useful in the practice of this invention, it is possible to employ not only elastase obtained by such an extraction technique but also that produced by synthetic or fermentation techniques. No special limitation should thus be imposed on elastase in terms of its origin or preparation technique. Certain characteristic features of elastase extracted and provided using swine pancreas as a raw material will be given next by way of example.

First of all, its molecular weight, isoelectric point, sedimentation coefficient and w are 25,900 (a value determined from the one-dimensional arrangement of amino acids), pH $9.5 \pm 0.5$, $S_{20}$ and 2.6, respectively. Turning next to its activities, it contains serine and histidine at its active sites and has such properties that in addition to elastin, it degrades singularly and significantly the methyl ester of N-α-benzoyl-L-alanine or acetyl-L-trialanine p-nitroanilide which is a synthetic substrate. According to measurement results obtained using the methyl ester of N-α-benzoyl-L-alanine as a substrate, the optimum pH for its activities ranges from 8 to 10 with 8.8 or so being particularly preferred. On the other hand, its activities are inhibited by NaCl, KCl, $(NH_4)_2SO_4$, NaCN and $CuSO_4$. Its activities are also inhibited by certain N-α-benzoylcarboxyl derivatives.

As defined at the beginning of this specification, the term "hair care" as used herein has a definition which should be interpreted in broad sense. As will become apparent from Experiment 1 which will be given herein, the term "hair care" means to promote the outgrowth of new hairs and to foster outgrown hairs and at the same time, embraces stimulation of scalp metabolism and hence prevention of dipilation. Hairs are formed by hair bulb cells. After their growth over a certain period of time, they enter the degeneration stage and will eventually be subjected to depilation. During this hair life cycle, elastase have such effects as stimulating the outgrowth of new hairs, promoting the growth of outgrown hairs, and inhibiting their prematured degeneration and depilation. Elastase has such hair care effects as defined by the combination of these effects. Therefore, the term "hair care" as used herein must be interpreted in the above-described concept.

Application of the hair care agent of this invention is effected principally by coating it directly to each intended site on the scalp. The hair care agent of this invention may however be formed into preparations suitable for its coating application in a principal embodiment of this invention, because it is most preferred to apply the hair care agent by its coating in order to draw out its effects promptly. The amount of elastase to be used and its toxicity will vary depending on the manner of its application. However, they may generally be as follows. When coated, the amount of elastase cannot be limited specifically because it varies considerably depending on the frequency of application and the density of hairs per unit area. It may instead be possible to define the concentration of elastase in the hair care agent of this invention. Its preferred concentration may be 0.001—1.0 w/v%. It should however be borne in mind that the present invention is by no means limited by or to this concentration range. When coated, no particular toxicity against the scalp was observed.

Elastase has such toxicity as summarized in the following table.

## Acute Toxicity $LD_{50}$ (EL.U./kg)

| Animal | Sex | Oral | Sub-cutaneous | Intraperi-toneal | Intra-venous |
|---|---|---|---|---|---|
| Rat | ♂ | >150,000 | >75,000 | 6,380 | 6,380 |
| Rat | ♀ | >150,000 | >75,000 | 5,850 | 6,380 |
| Mouse | ♂ | >150,000 | >75,000 | 4,970 | 5,100 |
| Mouse | ♀ | >150,000 | >75,000 | 2,780 | 4,310 |

Other toxicity will be shown in the following various experiments.

*Subacute toxicity:*

Elastase was orally administered at 750, 7,500, 37,500 and 75,000 EL.U./kg/day to male and female Wistar rats for straight 4 weeks and at 900 and 4,500 EL.U./kg/day to male and female beagles for straight 12 weeks.

As a result, no abnormal or pathogenic finding which is worth mentioning specifically was recognized in general appearance, blood and urine tests and morphological observation (both visual and histological).

*Chronic toxicity:*

Elastase was orally administered at 2,250, 5,700, 11,250 and 22,500 EL.U./kg/day to male and female Wistar rats for straight 24 weeks.

As a result, no abnormal or pathogenic finding which is worthy to mention specifically was recognized in general appearance, blood and urine tests and morphological observation (both visual and histological).

*Teratogenicity:*

In the organofaction stages of pregnant mice and rats, elastase was forcedly and orally administered at 750, 7,500 and 75,000 EL.U./kg/day to them for 6 days. As a result, there were observed neither death, growth suppression and teratogenic effects on their fetuses nor effects on morphological and functional differentiation of the neonates.

When the hair care agent of this invention is applied to the scalp, it is preferred to form it into a liquid lotion as a specific preparation form thereof. However, no problem or inconvenience will arise when it is formulated into a cream-like ointment. It should thus be borne in mind that the present invention is not limited by or to any specific preparation form. Although elastase is an essential ingredient in the hair care agent of this invention, other ingredients for the hair care agent of this invention, fat-soluble vitamins such as vitamin E, nicotinic acid and vitamin E, antihistaminic agents, hormones and/or vasodilators may be used. It should however be borne in mind that the present invention is not limited specifically by or to the incorporation of these optional ingredients. Each preparation form suitable for application to the scalp may be obtained by a method known *per se* in the art, using ingredients, required for the preparation of the corresponding preparation form, as materials.

The present invention will be described specifically by the following Examples.

Example 1

Silicone oil (30 g), polyoxyethylene-hardened castor oil (0.5 g) and a phosphate buffer of pH 7 (in an amount required to make the total volume to 1 liter) were added to 95% ethyl alcohol (950 ml). After dissolving the mixture into an intimate solution at ordinary temperature, elastase (2 g) was added with stirring to prepare a uniformly-suspended lotion as a scalp-coating hair care agent of this invention. It was filled in sealed containers and stored below 30°C. It was necessary to shake the containers prior to each application.

Example 2

Cholesterol (5 g), purified soybean lecithine (2 g), glycerin (30 g) and silicone oil (10 g) were added to 95% ethyl alcohol to make the total volume to 1 liter. After dissolving the former to form a homogeneous clear solution, elastase (1 g) was added with stirring to prepare a uniformly-suspended lotion as a scalp-coating hair care agent of this invention. It was filled in sealed containers and stored below 30°C. It was necessary to shake the containers prior to each application.

Example 3

Butoxypolyalkyleneglycol ether (150 g), Vancide® (1 g), polyoxyethyleneoleyl alcohol ether (15 g) and perfume (15 g) were uniformly dissolved in 95% ethyl alcohol (800 ml), followed by addition of propylene glycol (30 g) and a phosphate buffer of pH 6.5 (in an amount required to make the total volume to 1 liter) to obtain a clear solution. Elastase (1 g) was added with stirring to the solution to prepare a uniformly-suspended lotion as a scalp-coating hair care agent of this invention. It was filled in sealed containers and stored below 30°C. It was necessary to shake the containers prior to each application.

Effects of this invention will next be described by the following Experiment:

Experiment 1:

*Text materials:*

Provided as a test sample was a lotion prepared in accordance with the procedure described in Example 2. In addition, another lotion which had been prepared in the same manner as in Example 2 except for the omission of elastase was provided as a control.

*Method:*

As laboratory animals, Wistar rat groups (♂) of 5 months old and Wistar rat groups (♂) of 50 months old were used. Back hairs of each rat was removed by a depilatory and its back was washed thoroughly. Then, the test sample and control were coated every day in an amount of 0.5—1 ml per 10 cm² of the skin to its left half area and its right half area respectively. To the rat groups of 5 months old, the test sample and control were coated every day for 20 days. On the other hand, they were coated every day for 45 days to the rat groups of 50 months old. Hairs were then pulled out from both areas. Out of these hairs, the lengths of 100 hairs were measured. Measurement data obtained by repeating the above procedure twice or more were averaged.

*Results:*

Results are shown in Figs. 1 and 2. Namely, Figs. 1 and 2 are histograms showing the distribution in length of hairs on the backs of the rats under the experimental conditions. Hair lengths (mm) are plotted along the axis of abscissas, while the number of hairs in each length range is plotted along the axis of ordinates. In the drawings, plain rectangles indicate results on the control while hatched rectangles designates results on the test sample. Fig. 1 summarizes results obtained on the rat groups of 50 months old, while Fig. 2 summarizes results collected on the rat groups of 5 months old. It is clearly envisaged from Fig. 1, which pertains to the aged rats having extremely declined hair growth, that the hair care agent of this invention is effective in promoting the growth of such hairs by as much as about twice. In the case of the young rats featuring fast hair growth, it is understood from Fig. 2 that the hair care agent of this invention is also effective in promoting the growth of hair.

## Claims

1. A hair care agent for topical application comprising elastase as an effective ingredient.

2. A hair care agent as claimed in Claim 1, wherein the hair care agent is in a preparation form suitable for its application to the skin.

3. A hair care agent as claimed in Claim 2, wherein the content of elastase is 0.001—1.0 w/v%.

## Patentansprüche

1. Haarpflegemittel zur lokalen Anwendung, das Elastase als einen wirkstoff enthält.

2. Haarpflegemittel nach Anspruch 1, wobei das Haarpflegemittel in einer Zubereitungsform vorliegt, die für die Anwendung auf der Haut geeignet ist.

3. Haarpflegemittel nach Anspruch 2, wobei der Gehalt an Elastase bei 0,001 bis 1,0 Gew./Vol.% liegt.

## Revendications

1. Produit d'application locale pour les soins des cheveux comprenant de l'élastase comme ingrédient actif.

2. Produit pour les soins des cheveux tel que revendiqué dans la revendication 1, où le produit pour les soins des cheveux est sous forme d'une préparation approprièe à l'application sur la peau.

3. Produit pour les soins des cheveux tel que revendiqué dans la revendication 2, où la teneur en élastase est de 0,001 à 1,0% en p/v.

Fig. 1

Aged rats
Control
Sample

Number of hairs

Hair length (mm)

Fig. 2

Young rats

Control
Sample

Number of hairs

Hair length (mm)